Europäisches Patentamt

European Patent Office

Office européen des brevets

(19)

(11) Veröffentlichungsnummer: **0 509 203 A2**

## (12) EUROPÄISCHE PATENTANMELDUNG

(21) Anmeldenummer: **92102237.2**

(22) Anmeldetag: **11.02.92**

(51) Int. Cl.5: **C08G  63/08**, A61L 31/00,
A61K 7/40

(30) Priorität: **17.04.91 DE 4112489**

(43) Veröffentlichungstag der Anmeldung:
**21.10.92 Patentblatt  92/43**

(84) Benannte Vertragsstaaten:
**AT BE CH DK ES FR GB GR IT LI LU NL SE**

(71) Anmelder: **Jürgens, Christian, Dr. med.
Hochallee 19
W-2000 Hamburg 13(DE)**

(72) Erfinder: **Jürgens, Christian, Dr. med.
Hochallee 19
W-2000 Hamburg 13(DE)**

(74) Vertreter: **Siewers, Gescha, Dr.
Rechtsanwälte Dres. Harmsen, Utescher pp.
Adenauerallee 28
W-2000 Hamburg 1(DE)**

(54) **Resorbierbare, physiologisch unbedenkliche Copolymere und deren Verwendung.**

(57) Die Erfindung betrifft die Verwendung von Copolymeren aus razemischem Lactid und $\epsilon$-Caprolacton, $\delta$-Valerolacton, $\gamma$-Decalacton oder $\beta$-Hydroxybuttersäure, hergestellt durch die Umsetzung der Monomere im Molverhältnis Lactid zu Reaktionspartner von etwa 95 bis 70 zu 5 bis 30 unter Zugabe von an und für sich bekannten Metallcarboxylaten als Initiator bei Temperaturen von etwa 150°C über eine Zeitspanne von etwa 16 bis 18 Stunden, für die topische Behandlung menschlicher oder tierischer Haut.

EP 0 509 203 A2

Die Erfindung betrifft resorbierbare, physiologisch unbedenkliche Copolymere und deren Verwendung.

Es ist bekannt, daß hochpolymere Hydroxycarbonsäuren wie Polylactid und Polyklykolid physiologisch unbedenklich sind und eine hohe Körperverträglichkeit aufweisen, so daß sie bereits seit längerem in der Chirugie als langsam resorbierbare Fäden oder als Osteosyntheseimplantate eingesetzt werden. Diese hochpolymeren Polycarbonsäuren werden im Verlauf von Wochen bis Monaten abgebaut und vom menschlichen oder tierischen Körper in üblicher Weise über den Zitronensäurezyklus oder den Fettstoffwechsel abgebaut. Aus diesem Grund können beispeilsweise derartige Polymere auch unbedenklich als Osteosyntheseimplantate benutzt werden, wobei sich noch der Vorteil ergibt, daß die Sekundäroperation zur Entfernung der Implantate nicht mehr oder nur noch für den nicht aus Polymer bestehenden Teil des Implanteates notwendig ist.

Außerdem sind aus der DE-OS 36 20 685 Mittel auf der Basis von fließfähigem bis festen oligomeren Estern der Milchsäure und/oder Glykolsäure zur Abdeckung unverletzter und/oder verletzter menschlicher oder tierischer Haut bekannt. Diese Zubereitungen bestehen im wesentlichen aus den oligomeren Estern als resorbierbarem Träger oder Filmbilder und können zusätzlich hautpflegende, regenerierende, desinfizierende oder das Epithel stimulierende Substanzen enthalten. Außerdem sind aus der FR-OS 21 26 270 filmbildende Polymere aus Milchsäure und Glykolsäure in Lösungsmitteln wie beispielsweise Ethylacetat bekannt, die zusätzlich noch pharmakologische Wirkstoffe enthalten und beispielsweise als Sprühverband eingesetzt werden können.

Außerdem sind aus der US-PS 4,045,418 oder 4,148,871 Copolymere aus Lactid und ε -Caprolacton bekannt, die physiologisch unbedenklich sind und beispielsweise als subdermal einpflanzbare Behältnisse für Arzneimittel eingesetzt werden können. Über die Verträglichkeit und die biologische Abbaubarkeit solcher Behältnisse wird beispielsweise in Journal of Biomedical Materials Research, Band 13, Seiten 497 bis 507, 1979 und in Naltrexone: Research Monograph 28, National Institute on Drug Abbusus, 1983 von der Arbeitsgruppe Colin G. Pitt et al. referiert.

Nachteilig an den bisher bekannten Mono- und Copolymeren aus Milchsäure und Glykolsäure ist die Tatsache, daß es sich um Verbindungen handelt, die in der Regel in Lösungen aufgetragen werden und die von sich aus relativ hart und wenig flexibel sind, so daß derartige aufgesprühte Filme nach dem Verdunsten des Lösungsmittels schnell brüchig und rissig werden, wobei noch hinzukommt, daß die Anhaftung auf Haut- oder Wundfläche nicht besonders gut ist. Die bisher verwendeten Polymere aus Lactid und ε-Caprolacton andererseits sind in der Regel steife Thermoplasten, die sich beispielsweise zur Herstellung von Behältnissen eignen, aber nicht für eine topische Anwendung geeignet waren.

Es besteht daher immer noch ein Bedarf nach physiologisch unbedenklichen auf der Haut verwendbaren Polymeren, die die geschilderten Nachteile nicht aufweisen.

Erfindungsgemäß wird nun die Verwendung von Copolymeren aus razemischem Lactid und ε-Caprolacton, δ-Valerolacton, razemischem γ-Decalacton oder β-Hydroxybuttersäure vorgeschlagen, wobei die Copolymeren durch Umsetzung der Monomere im Molverhältnis Lactid zu Reaktionspartner von etwa 95 bis 70 zu 5 bis 30 unter Zugabe von an sich bekannten Metallcarboxylaten als Initiator bei Temperaturen von etwa 150°C während einer Zeitspanne von etwa 16 bis 48 Stunden hergestellt werden.

Die erdindungsgemäßen Copolymere sind der Kettenlänge nach als Poly- und Oligomere anzusehen und liegen als Gemisch aus Mono-, Oligo- und Polymeren vor. Es handelt sich um farblose transparente Verbindungen, die je nach dem Molverhältnis der Monomeranteile zäh verfließlich bis starr sind. Hergestellt werden die beanspruchten Verbindungen durch Umsetzen der Monomere im Molverhältnis von Lactid zu Reaktionspartner von etwa 95 bis 70 zu 5 zu 30, wobei die Erhöhung des Lactidanteiles zu einer Erhöhung einer Erweichungstemperatur führt. Die Umsetzung erfolgt unter Zugabe von an und für sich bekannten Metallcarboxylaten als Initiator bei Temperaturen um etwa 150°C während einer Zeitdauer von etwa 16 bis 48 Stunden. Das Verhältnis von Reaktionsmischung zu Initiator beträgt etwa 100 zu 1 bis 500 zu 1, wobei bei Erhöhung dieses Verhältnisses bei der Reaktion der Anteil an längeren Molekülketten erhöht wird und dadurch auch eine Erhöhung des Erweichungspunktes bedingt ist. Als Initiator wird vorzugsweise Zinn-II-diethylhexanoat eingesetzt, da sich herausgestellt hat, daß andere an und für sich bekannte Initiatoren in der Regel eine geringere Ausbeute ergeben oder zu Trübung/Färbung führen. Außerdem wurde gefunden, daß eine Temperatur von 150°C und eine Umsetzungszeit von 16 bis 48 Stunden nicht überschritten werden soll, da es durch zu langes Erhitzen bereits zu einem beginnenden Abbau des Polymeres kommen kann. Die Reaktion wird nach der gewünschten Umsetzungsdauer entweder durch Abkühlung der Umsetzungsmischung oder durch Zugabe von beipielsweise Chelatisierungsmitteln abgebrochen. Zur Entfernung von verbleibenden Monomeren, kurzkettigen Oligomeren oder ggf. auch überschüssigen Weichmachern wird die Reaktionsmasse in der Regel mit der 600 bis 800-fachen Menge Alkohol ausgefällt; ggf. kann die Reaktionsmasse auch mit Wasser oder einer wäßrigen Lösung ausgewaschen werden, da sich hierdurch auch bereits ein großer Teil von Monomeren, kurzkettigen Oligomeren oder Weichmachern entfernen läßt.

Falls erwünscht, können der Reaktionsmasse Weichmacher zugesetzt werden, um die Erweichungstemperatur und die Diffundibilität zu verändern. Als Weichmacher werden vorzugsweise überschüssiges Caprolacton, Tributylcitrat oder Phthalsäureester verwendet. Der Anteil an Weichmachern sollte in der Regel 10 bis 20%, bezogen auf das Gewicht, nicht überschreiten.

Die erfindungsgemäßen Copolymere können in geeigneten organischen Lösungsmitteln wie beispielsweise Essigester, Aceton, Methylenchlorid oder THF in Lösung gebracht werden. Sie lassen sich ohne weiteren Zusatz zu Folien auswalzen, die allerdings beispielsweise auch durch Verdunstung hergestellt werden können. Andere für Kunststoffe geeignete Verarbeitungsmethoden sind ebenfalls anwendbar.

Sowohl die Monomere als auch die Polymere werden in vivo und in vitro durch Hydrolyse abgebaut. Die Copolymere und ihre Abbauprodukte sind medizinisch unbedenklich und nicht allergen. Die Monomere werden in vivo über den Zitronensäurezyklus bzw. über den Fettsäurestoffwechsel weiter metabolisiert. Es hat sich herausgestellt, daß die Zeiten für den hydrolytischen Abbau um so kürzer sind, je höher der Lactidanteil liegt.

Die erfindungsgemäß beanspruchten Polymere können für vielfältige medizinische und kosmetische Zwecke eingesetzt werden. Ein Haupteinsatzgebiet ist die Verwendung als chirurgische Operationsinzisionsfolie. Die in Lösung in einem geeigneten Lösungsmittel wie Methylenchlorid, Aceton oder Essigester vorliegenden Verbindungen lassen sich ohne weiteres mit bekannten Desinfektionsmitteln versetzen; auch ist eine Kombination mit lokal wirkenden Anaesthetika möglich. Bei Verwendung von Essigester ist eine desinfizierende Wirkung bereits aufgrund dieses Lösungsmittels gegeben.

Für topische Verwendungen am geeigentsten sind Copolymere mit einem Molverhältnis zwischen 70 zu 30 bis 85 zu 15 und einem Verhältnis von Reaktionspartner zu Initiator von etwa 300 zu 1 bis 500 zu 1 und einer Polymerisationszeit von 24 Stunden. Hierbei handelt es sich um gewaschene Copolymere; aber auch ungewaschene Copolymere mit Molverhältnissen zwischen 80 zu 20 bis 90 zu 10 und einem Verhältnis von Reaktionspartner zu Initiator von 100 zu 1 bis 400 zu 1 bei gleicher Polymerisationszeit sind gut verwendbar. Dieselben Verbindungen lassen sich in gleicher Weise für Inzisionsfolien einsetzen, wobei der große Vorzug vor allen Dingen in der Umweltfreundlichkeit, der einfachen Applikation, der Unbegrenztheit der abzudeckenden Flächen und den geringen Kosten der Herstellung und Aufbereitung liegt, wobei hinzukommt, daß im Gegensatz zu Lactid-Glykolid-Polymeren eine wesentlich verbesserte Anhaftung auf Haut- und Wundfläche und eine hohe Flexibilität und Modellierbarkeit festzustellen ist. Außerdem fallen die erfindungsgemäßen Zubereitungen durch die geringe Ablösbarkeit durch wäßrige Flüssigkeiten wie Blut, Lymphe usw. auf. Der natürliche hydrolytische Abbau der Sprühfolie beansprucht etwa 3-30 Wochen in vitro.

Weiterhin können die erfindungsgemäßen Mittel besonders günstig als flüssiger Handschuh beim Umgang mit Allergenen eingesetzt werden. So sind sie beispeilsweise auch für Allergiker bei Spül- oder Waschmittelallergien eine echte Alternative. Aufgrund der langen Haltbarkeit und der guten Haftung können derartige Lösungen auch als Hilfsmittel bei der Camouflage Verwendung finden und insbesondere bei Kombinationen mit Sonnenschutzfaktoren als langanhaftender Sonnenschutz, der beispielsweise das erneute Auftragen nach dem Schwimmen tagelang unnötig macht.

Die Erfindung wird im folgenden anhand der Beispiele näher erläutert:

Beipiel 1

Herstellung der Copolymere

Zur Herstellung der Copolymere werden D L-Lactid und $\epsilon$-Caprolacton in einem Molverhältnis von 85 zu 15, entsprechend 70,2 Gramm zu 9,8 Gramm langsam auf 150°C erhitzt. Sodann wird als Polymerisationsinitiator 1,16 ml Zinn-II-diethylhexanoat (Verhältnis Reaktionsmasse zu Initiator = 100 zu 1) zugegeben. Die Polymerisation erfolgt über 24 Stunden bei 150°C im Ölbad. Anschließend wird die Mischung abgekühlt und bei 70°C mit Ethylacetat auf 1 Liter aufgefüllt. Diese Lösung verbleibt dann für 36 Stunden auf einer Schüttelmaschine und kann danach als solche verwendet werden.

In entsprechender Weise und Berücksichtigung der Molverhältnisse lassen sich auch die Copolymeren von Lactid und den anderen Lactonen wie Valerolacton, Heptalacton, Decalacton oder auch auch von $\beta$-Hydroxysäuren wie $\beta$-Hydroxybuttersäure herstellen.

Beispiel 2

Herstellung einer Operationsinzisionsfolie

D, L-Lactid und $\epsilon$-Caprolacton werden in einem Molverhältnis von 90 zu 10 entsprechend 73,5 Gramm zu 6,5 Gramm langsam auf 150°C erhitzt.

Durch Zugabe von 0,6 ml Zinn-II-diethylhexanoat (Verhältnis von Reaktionsmasse zu Initiator = 100 zu 1) wird die Polymerisation initiiert. Die Masse wird dann 24 Stunden bei 150°C im Ölbad erhitzt. Anschließend wird die Masse abgekühlt und kann bei 70°c mit Essigester auf 1 Liter aufgefüllt und 36 Stunden auf einer Schüttelmaschine behandelt werden. Die fertige Lösung kann dann direkt als flüssiger Spray eingesetzt werden.

In gleicher Weise können für die Verwendung als Sonnenschutzspray oder als Schminkgrundlage die entsprechend hergestellten Lösungen Einsatz finden. Bei der Verwendung als Sonnenschutz werden der fertigen Lösung die an und für sich bekannten und üblichen UVA- und UVB-Filter beigemischt.

Beispiel 3

Herstellung eines flüssigen Handschuhs

Die nach Beispiel 1 hergestellte Lösung wird in an und für sich bekannte Pumpsprays oder Aerosolsprays abgefüllt.

**Patentansprüche**

1. Verwendung von Copolymeren aus rezemischem Lactid und $\epsilon$-Caprolacton, $\delta$-Valerolactont, $\gamma$-Decalacton oder $\beta$-Hydroxybuttersäure, hergestellt durch die Umsetzung der Monomere im Molverhältnis Lactid zu Re- aktionspartner von etwa 95 bis 70 zu 5 bis 30 unter Zugabe von an und für sich bekannten Metallcarboxylaten als Ini- tiator bei Temperaturen von etwa 150°C über eine Zeitspan- ne von etwa 16 bis 18 Stunden, für die topische Behandlung menschlicher oder tierischer Haut.

2. Verwendung der Copolymere nach Anspruch 1 als Operationsinzisionsfolie.

3. Verwendung der Copolymere nach Anspruch 1 als flüssiger Handschuh

4. Verwendung der Copolymere nach Anspruch 1 als Sonnenschutzmittel.

5. Verwendung ver Copolymere nach Anspruch 1 als Hilfsmittel bei der Camouflage.